# EUROPEAN PATENT SPECIFICATION

(11) **EP 3 365 038 B1**
(45) Date of publication and mention of the grant of the patent: **03.07.2019**
(21) Application number: 16784969.4
(22) Date of filing: 06.10.2016
(51) Int. Cl.: A61L 31/06, A61L 31/16, A61L 15/26, A61L 15/32, A61L 15/38, A61L 15/58, A61L 15/64, A61L 24/10

(54) **IMPROVED FIBRINOGEN-BASED TISSUE ADHESIVE PATCH**
VERBESSERTES GEWEBEHEFTPFLASTER AUF DER BASIS VON FIBRINOGEN
TIMBRE ADHÉSIF POUR TISSU À BASE DE FIBRINOGÈNE AMÉLIORÉ

(30) Priority: 19.10.2015 US 201562243158 P
(43) Date of publication of application: 29.08.2018
(73) Proprietor: Sealantium Medical Ltd, 4809239 Rosh Ha'Ayin (IL)
(72) Inventor: LAUB, Orgad, 69626 Tel Aviv (IL); COHEN, Eran, 4946410 Petah-Tiqva (IL); SCHWARTZ, Yotam, 7575122 Rishon LeZion (IL)
(74) Representative: Puchberger & Partner Patentanwälte
(86) International application number: PCT/IL2016/051090
(87) International publication number: WO 2017/068572

(56) References cited:
- EP-A2- 0 334 998
- EP-A2- 1 093 824
- WO-A1-99/21908
- WO-A1-2014/174509

## Description

### FIELD OF THE INVENTION

This invention relates in general to tissue sealant adhesive patches. In particular, it relates to improved patches in which fibrinogen-based tissue adhesive patches are produced using technology adapted from the coating industry.

### BACKGROUND OF THE INVENTION

Wound dressings, tissue coatings, and tissue adhesives are examples of devices that serve to stop or prevent leakage of blood and other bodily fluids. These dressings can serve to seal open wounds, prevent infection, and so on. Many types of wound dressings and tissue adhesives known in the literature incorporate one or more coagulants such as fibrinogen.

Numerous examples are known in the literature of coagulant-containing tissue sealant compositions. U.S. Pat. No. 5631011 discloses a tissue treatment composition comprising fibrin or fibrinogen and a polymer that is biodegradable and biocompatible. The composition acts as a glue to bind tissue, e.g. a cut and sutured blood vessel. U.S. Pat. No. 6699844 discloses a fibrin-containing tissue sealant that also contains a derivative of hyaluronic acid. U.S. Pat. No. 6162241 discloses a hemostatic tissue sealant comprising a biocompatible, biodegradable hydrogel tissue sealant comprising crosslinkable groups having incorporated therein an effective amount of a hemostatic agent to stop the flow of blood from tissue in a medically acceptable period of time. U.S. Pat. No. 6056970 discloses compositions, produced by known paper-making technology, that comprise hemostatic compounds and bioabsorbable polymers.

Methods are also known in the art for preparing compositions that can release a pharmaceutically effective agent such as a hemostatic agent from a polymeric matrix. For example, U.S. Pat. No. 6194005 discloses a method in which a powdered pharmaceutically effective agent is sprayed onto a warm lipid matrix, which thereby coats the agent. U.S. Pat. No. 6579537 discloses a method for producing inter alia a fibrinogen composition using a polyalkylene glycol. The basic method comprises producing a solution of fibrinogen and fibronectin and precipitating the fibrinogen and fibronectin by adding a polyalkylene glycol and an amino acid. U.S. Pat. Appl. Pub. No. 2012/0121532 discloses a method for preparing a dry and stable hemostatic composition. A dry hemostatic agent is mixed with a dry polymeric component in proportions such that on addition of an appropriate diluent (e.g. water), a polymeric matrix (e.g. a hydrogel) into which the hemostatic agent is incorporated.

Also known in the art are non-fibrous polymer films or coatings that incorporate a hemostatic agent such as thrombin. For example, U.S. Pat. Appl. Pub. No. 2007/0059346 discloses a film containing nitroglycerin and possibly other therapeutic agents; the film is made of a water-soluble polymer that can dissolve in the mouth of a patient.

Hemostatic wound dressings that incorporate fibrinogen are also known in the art. U.S. Pat. No. 7189410 discloses a layered fibrin sealant bandage comprising a backing layer and a hemostatic component layer containing fibrinogen, the fibrinogen acting to produce a clot when the bandage is applied to a wound. A family of patents that includes inter alia U.S. Pat. No. 6054122 discloses fibrin sealant bandages that comprise an occlusive backing, an adhesive layer on the wound-facing surface of the backing, and a layer of dry hemostatic materials (fibrinogen, thrombin, and Ca²⁺ and/or Factor XIII as necessary). The dry materials adhere to, but are not incorporated into, the adhesive layer and are exposed at the time of use. U.S. Pat. Appl. Pub. No. 2006/0155235 discloses a hemostatic compression bandage that bandage comprises a flexible backing element, a powdered hemostatic substance, and a flexible film element. In this bandage, the hemostatic substance remains as a free powder. Immediately prior to use, the flexible film element is peeled away, exposing the powder, which is then placed directly on the wound. U.S. Pat. Appl. Pub. No. 2012/0070485 discloses a patch comprising a fibrin nanofiber mesh.

The present inventors have recently disclosed, in PCT Pat. Appl. Pub. No. WO2014/174509 (henceforth **'509**) fibrinogen-based tissue adhesive patches in which a fibrin sealant is incorporated into a polymer film. In contrast to those known in the art, the patches disclosed in '509 do not have any mesh or woven component, and use the fibrin sealant only to attach the patch to the tissue, the sealing of the tissue being performed by the polymer film. These patches thus provide a significant savings in material and ease of use.

Ideally, a tissue adhesive patch would remain intact long enough to stop bleeding or leakage of fluid from the tissue being sealed, but would decompose or degrade rapidly thereafter in order to minimize tissue irritation. The patches disclosed in **'509** remain intact for more than two weeks, which is longer than necessary for some applications, in which a decomposition time on the order of days would be preferable. There is therefore a long-felt but as yet unmet need for an improved tissue adhesive patch that retains the advantages of the patches disclosed in **'509,** but that has a faster degradation time.

### SUMMARY OF THE INVENTION

The scope of the invention is defined by the claims. Any references in the description to methods of treatment refer to the pharmaceutical compositions, respectively patches of the present invention for use in a method for treatment of the human (or animal) body by therapy.

The tissue adhesive patches disclosed in the present invention are designed to meet this need. In particular, the inventors have discovered that, surprisingly, the critical parameter that determines the half-life of the film is the ratio of the hydrophilic to hydrophobic components of the biocompatible polymers that make up the polymer film and that the degradation time of the patch is a sensitive function of this ratio.

It is therefore an object of the present invention to disclose a fibrinogen-based tissue adhesive patch, wherein said adhesive patch comprises: a backing made from a film made of a biocompatible polyethylene glycol-caprolactone-lactide (PEG-CL-LA) triblock copolymer (PECALA), wherein said PECALA comprises PEG having a molecular weight of between 3000 and 3500 and a CL:LA ratio of 34:2; and a fibrinogen sealant incorporated into said biocompatible polymer backing; wherein said fibrinogen sealant comprises less than 8 mg/cm² fibrinogen and less than 20 IU/cm² thrombin. In preferred embodiments of the invention, said fibrinogen sealant comprises about 2 mg/cm² fibrinogen and 10 IU/cm² thrombin. In more preferred embodiments of the invention, said fibrinogen sealant comprises less than 8 mg/cm² fibrinogen, less than 20 IU/cm² thrombin, and CaCl₂. In some preferred embodiments of the invention, said fibrinogen sealant consists of less than 8 mg/cm² fibrinogen, less than 20 IU/cm² thrombin, and CaCl₂. In some particularly preferred embodiments of the invention, said fibrinogen sealant consists of about 2 mg/cm² fibrinogen, about 10 IU/cm² thrombin, and CaCl₂.

It is the object of this invention to disclose the fibrinogen-based tissue adhesive patch as defined in any of the above, wherein said PECALA comprises PEG having a molecular weight of between 3000 and 3500 and a CL:LA ratio of 34:2.

It is a further object to disclose the fibrinogen-based tissue adhesive patch as defined in any of the above, wherein at least one parameter characterizing said PECALA is chosen so as to provide said patch with a predetermined degradation time. In some embodiments, said at least one parameter is selected from the group consisting of PEG molecular weight; ratio of hydrophilic to hydrophobic components; CL:LA ratio; and crystallinity. In some preferred embodiments of the invention, said predetermined degradation time is no more than two weeks. In some particularly preferred embodiments of the invention, said predetermined degradation time is between 10 and 14 days.

It is a further object of this invention to disclose the fibrinogen-based tissue adhesive patch as defined in any of the above, wherein said patch is characterized by a thickness of between 130 nm and 170 nm.

It is a further object of this invention to disclose the fibrinogen-based tissue adhesive patch as defined in any of the above, wherein said fibrinogen sealant additionally comprises at least one additive. In some embodiments of the invention, said additive is selected from the group consisting of additives for extending the adhesion half-life of said film, pharmaceutically active agents, and analgesics. In some embodiments of the invention, said additive is a plasmin inhibitor for extending the adhesion half-life of said film. In some embodiments of the invention, said additive is a pharmaceutically active agent for targeted or controlled release.

It is a further object of this invention to disclose a method for producing a fibrinogen-based tissue adhesive patch, wherein said method comprises: casting a polymer film from PECALA, thereby creating a polymer film characterized by a thickness; softening said polymer film; placing a fibrinogen sealant comprising less than 8 mg/cm² fibrinogen and less than 20 IU/cm² thrombin on at least one surface of said polymer film; and, pressing said polymer film until at least a portion of said fibrinogen sealant is incorporated into the surface of said polymer film; wherein said PECALA comprises PEG having a molecular weight of between 3000 and 3500 and a CL:LA ratio of 34:2.

It is a further object of this invention to disclose a method for preparing a fibrinogen-based tissue adhesive patch, wherein said method comprises: heating to a predetermined temperature a work surface in connection with a source of vacuum; engaging said vacuum to said work surface; applying a solution of PECALA to said work surface; adjusting a polymer blade to a predetermined height above said work surface; spreading said solution of PECALA over said work surface with said polymer blade; evaporating said solvent, thereby creating a non-permeable biocompatible polymer film characterized by a thickness; heating said work surface above said softening temperature; spreading over said polymer film a powder comprising a fibrinogen sealant, said fibrinogen sealant comprising less than 8 mg/cm² fibrinogen and less than 20 IU/cm² thrombin; placing over said polymer film a top release sheet over said powder and polymer film; applying pressure to said top release sheet so as to at least partially incorporate said powder into said polymer film, thereby forming a film of adhesive patch material; removing said top release sheet from said film of adhesive patch material; releasing said vacuum; cooling said work surface to room temperature; and, removing said adhesive patch material from said work surface; wherein said PECALA comprises PEG having a molecular weight of between 3000 and 3500 and a CL:LA ratio of 34:2.

It is a further object of this invention to disclose the method as defined in any of the above, wherein said fibrinogen sealant comprises less than 8 mg/cm² fibrinogen, less than 20 IU/cm² thrombin, and CaCl₂.

It is a further object of this invention to disclose the method as defined in any of the above, wherein said thickness is between 130 nm and 170 nm.

It is a further object of this invention to disclose the fibrinogen-based tissue adhesive patches for use in a method of treating a leak of fluid into or out of a body part, comprising applying a tissue adhesive patch as defined in any of the above to said body part, thereby causing said tissue adhesive patch to adhere to said body part, thereby sealing said body part. In some embodiments of the method, said body part is an artery or organ. In some embodiments of the invention, said leak of fluid is selected from the group consisting of arterial bleeding; organ tissue bleeding; bile anastomosis; cerebrospinal fluid leak; dura leak; and air leak in damaged lung tissue. In some embodiments of the method, said step of applying a tissue adhesive patch comprises manually pressing said patch on the surface of said body part.

It is a further object of this invention to disclose the fibrinogen-adhesive tissue adhesive patch according to the invention for use in the treatment of a leak of fluid into or out of a body part. In some embodiments of the invention, the tissue adhesive patch as defined in any of the above is for use in the treatment of a leak of fluid into or out of an artery or organ. In some embodiments of the invention, said leak of fluid is selected from the group consisting of arterial bleeding; organ tissue bleeding; bile anastomosis; cerebrospinal fluid leak; dura leak; and air leak in damaged lung tissue. In some preferred embodiments of the invention, said treatment comprises applying a tissue adhesive patch by manually pressing said patch on the surface of said body part.

### BRIEF DESCRIPTION OF THE DRAWINGS

The invention will now be described with reference to the drawings, in which
FIG. **1** contrasts the failure mode of the patches of the present invention with that of patches known in the art;
FIG. **2** presents a graph illustrating the adhesive strength of the patches of the present invention as a function of concentration of fibrin sealant; and,
FIG. **3** presents a graph illustrating the degradation times of patches of the present invention made with different formulations of the polymer film.

### DETAILED DESCRIPTION OF THE PREFERRED EMBODIMENTS

In the following description, various aspects of the invention will be described. For the purposes of explanation, specific details are set forth in order to provide a thorough understanding of the invention. Therefore the invention is not limited by that which is illustrated in the figure and described in the specification, but only as indicated in the accompanying claims, with the proper scope determined only by the broadest interpretation of said claims.

The following abbreviations are used throughout this application:
"PEG" is used to refer to polyethylene glycol.
"CL" is used to refer to caprolactone.
"LA" is used to refer to lactide.
"PECALA" is used to refer to a triblock copolymer comprising polyethylene glycol (PEG), caprolactone (CL), and lactide (LA) components. When the term PECALA is followed by two numbers, the first indicates the ratio of hydrophilic (PEG) to hydrophobic (CL and LA) repeat units, and the second to the number of lactide units per triblock flank.

As used herein, the term "about," when applied to numerical quantities, refers to a range of ±25 % of the nominal value.

As used herein, with reference to a polymer film or hemostatic patch attached to tissue, the terms "degrade" and "degradation" refer to the breakup of the polymer film or hemostatic patch into smaller pieces.

In **'509,** the present inventors disclosed hemostatic patches that contain a fibrinogen component that acts to attach the polymer film to the tissue and in which the polymer film itself rather than the fibrinogen component acts to seal the tissue. In contrast to hemostatic patches and dressings known in the art, these patches do not include a mesh or woven component, woven or non-woven fabrics, or materials made by techniques known in paper-making technology. Rather, these patches comprise a single layer of polymer film into which fibrinogen and thrombin are incorporated, in contrast to multilayer hemostatic dressings known in the art (although embodiments in which additional layers are added for ease of handling or storage are not excluded from the scope of the present invention). Furthermore, the fibrinogen sealant component is physically incorporated into the polymer film to form a single integrated unit, in contrast to those hemostatic patches and dressings known in the art in which the coagulant is present as a free powder.

The patches disclosed in **'509** generally retain their mechanical integrity for a period of several weeks following their attachment to the tissue. For some uses, however, the patch would ideally degrade on a faster time scale. For example, in the case of bleeding, the patch might not need to remain in place for more than a few days. Ideally, the patch would retain its mechanical integrity just long enough to complete the necessary treatment and would then degrade within a few days thereafter in order to minimize the possibility of negative interactions between the patch and the patient's body.

The present inventors have discovered, surprisingly, that for PECALA-based patches, several parameters can be varied in order to set a desired degradation time, and that patches with degradation times of on the order of two weeks can easily be produced. One important parameter is the ratio of the components of the triblock copolymer (e.g. the CL:LA ratio or ratio of hydrophobic to hydrophilic components). In general, the more CL units per PEG unit, the longer the degradation time, while the more CA units per PEG unit, the shorter degradation time (see the Example below). The degradation time is also apparently controlled by the crystallinity of the polymer; without wishing to be bound by theory, it appears that small amounts of LA prevent the CL from crystallizing, but too high a fraction of LA will itself crystallize, changing the degradation time. The molecular weight of the PEG used can also affect the degradation time. Thus, by proper control of the relative amounts of the components of the PECALA, it is possible to fine-tune the degradation time to the desired length.

According to the invention is a PECALA film comprising PEG of MW between 3000 and 3500 and a CL:LA ratio of 34:2 (i.e. 4 LA units and 68 CL units per PEG) into which a fibrinogen sealant comprising fibrinogen (≤ 8 mg/cm², preferably about 2 mg/cm²) and thrombin (≤ 20 IU /cm², preferably about 10 mg/cm²) has been incorporated. In preferred embodiments, the fibrinogen sealant also comprises CaCl₂. It may also include additives such as additives for extending the adhesion half-life of said film, pharmaceutically active agents, and analgesics.

In some embodiments, the patch thickness is about 200 µm; in preferred embodiments, the patches are about an order of magnitude thinner (typically 130 - 170 nm).

The patches may be prepared according to any method known in the art. For example, they can be prepared by the method disclosed in **'509.** In this method, a PECALA film is cast on a surface such as a glass slide from a solution of PECALA in a volatile organic solvent. The film is then heated to its softening point, and a powdered fibrin sealant mixture containing fibrinogen, thrombin, and CaCl₂ sprinkled onto the surface of the softened polymer film. In typical embodiments, the fibrin sealant mixture has been micronized to a particle size of 25 - 75 nm. The sealant mixture is then pressed into the surface of the softened polymer film and allowed to cool to room temperature. The film is then optionally placed in a freezer (typically at about -20 °C) to aid in removing it from the surface on which it was prepared. Excess powder is removed from the patch by shaking and the patch then removed from the surface on which it was prepared.

The inventors have recently developed a second process for manufacture of the patches. This method uses a specially modified drawdown coater, and comprises: (a) heating to a predetermined temperature a work surface in connection with a source of vacuum; (b) engaging said vacuum to said work surface; (c) applying a solution to said work surface, said solution comprising a biocompatible polymer characterized by a softening temperature dissolved in a solvent; (d) adjusting a polymer blade to a predetermined height above said work surface; (e) spreading said solution over said work surface with said polymer blade; (f) evaporating said solvent, thereby creating a non-permeable biocompatible polymer film; (g) heating said work surface above said softening temperature; (h) spreading a powder comprising a fibrinogen sealant over said polymer film; (i) placing over said polymer film a top release sheet over said powder and polymer film; (j) applying pressure to said top release sheet so as to at least partially incorporate said powder into said polymer film, thereby forming a film of adhesive patch material; (k) removing said top release sheet from said film of adhesive patch material; (1) releasing said vacuum; (m) cooling said work surface to room temperature; and, (n) removing said adhesive patch material from said work surface.

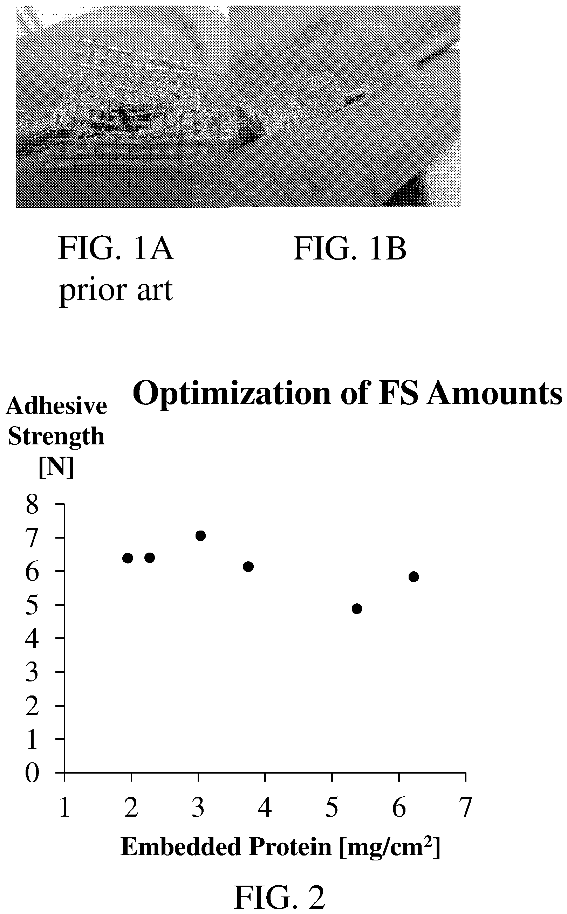

In typical embodiments of the patch, a force of about 5 - 7 N is required in order to detach it from tissue. Reference is now made to FIG. **1****,** which illustrates the failure mode of the patches of the present invention. FIG. **1A** illustrates a mesh embedded with fibrin of a type known in the art. When a detachment force is applied, the mesh is detached from the tissue and only residues of fibrin remain on the mesh, with no evidence of tissue fragments seen on the mesh. This behavior indicates that hemostatic patches known in the art undergo cohesive failure, i.e. the adhesive itself loses its structural integrity, fragments, and breaks.

In contrast, as shown in FIG. **1B****,** when a detachment force is applied to the patches of the present invention, the film detaches with fragments of tissue remaining on the film, indicating that the tissue itself rather than the adhesive underwent mechanical failure. That is, in contrast to hemostatic patches known in the art, the patches of the present invention undergo adhesive failure along the interface between the adhesive and the substrate. Thus, the instant invention comprises patches in which the main contribution to the sealing ability arises from the adhesive strength of the polymer film rather than the rather weak internal strength of fibrin.

Reference is now made to FIG. **2****,** which presents a graph showing the adhesive strength of the patches of the present invention as a function of the concentration of fibrin sealant. As can be seen from the figure, the adhesive strength is essentially independent of the amount of fibrin present. Thus, the patches of the present invention are effective with significantly less fibrin sealant than is used in patches known in the art. In preferred embodiments of the present invention, the fibrin sealant comprises about 2 mg/cm² fibrin and 10 IU/cm² thrombin.

### EXAMPLE

The following example provides an illustration of a preferred embodiment of the present invention in order to assist one of ordinary skill in the art to make and use the invention, and is not intended to be limiting in any way.

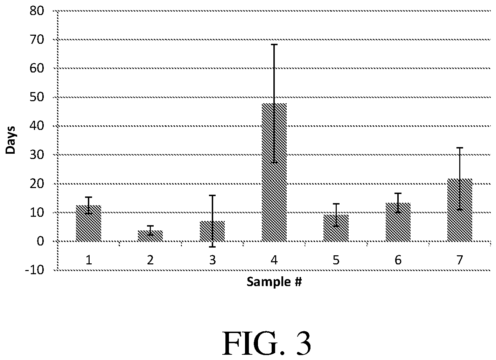

A series of hemostatic patches of the present invention were prepared with different PECALA formulations. The PECALA was prepared according to standard literature procedures. The degradation time of the patches was measured for a minimum of five independent samples. The results of the experiments are summarized in Table 1, and illustrated graphically in FIG. **3****.** Samples 2-5 and 7 are comparatives and not according to the invention.

**TABLE 1**

| **Sample** | **PEG MW** | **CL:LA ratio** | **Mean degradation time (days)** |
|---|---|---|---|
| 1 | 3350 | 34:2 | 12.5 |
| 2 | 3350 | 44:3 | 3.8 |
| 3 | 3350 | 44:4 | 7.0 |
| 4 | 3350 | 34:1 | 47.8 |
| 5 | 6000 | 34:2 | 9.2 |
| 6 | 3000 | 34:2 | 13.4 |
| 7 | 3350 | 40:2 | 21.7 |

In the table, the CL:LA ratio is given relative to the number of PEG units; that is, a CL:LA ratio of n:m indicates that for each PEG unit in the polymer, there were 2n CL units and 2m LA units.

As can be seen from the table, in general, the degradation time tended to decrease with increasing PEG molecular weight, decreasing CL:LA ratio, increasing number of CL units per PEG unit, and decreasing number of LA units per PEG unit. Because the degradation time appears to be a function of polymer crystallinity as well, these general rules are only valid within limits; for example, increasing the number of CL units per LA unit will increase the crystallinity of the polymer.

## Claims

1. A fibrinogen-based tissue adhesive patch, wherein said adhesive patch comprises:
a backing made from a film made of a biocompatible polyethylene glycol-caprolactone-lactide (PEG-CL-LA) triblock copolymer (PECALA); and,
a fibrinogen sealant comprising less than 8 mg/cm² fibrinogen and less than 20 IU/cm² thrombin incorporated into said biocompatible polymer backing;
wherein said PECALA comprises PEG having a molecular weight of between 3000 and 3500 and a CL:LA ratio of 34:2.

2. The fibrinogen-based tissue adhesive patch according to claim **1,** wherein said fibrinogen sealant comprises about 2 mg/cm² fibrinogen and 10 IU/cm² thrombin.

3. The fibrinogen-based tissue adhesive patch according to claim **1,** wherein said fibrinogen sealant comprises less than 8 mg/cm² fibrinogen, less than 20 IU/cm² thrombin, and CaCl₂.

4. The fibrinogen-based tissue adhesive patch according to any one of claims **1 - 3,** wherein at least one parameter characterizing said PECALA is chosen so as to provide said patch with a predetermined degradation time.

5. The fibrinogen-based tissue adhesive patch according to claim **4,** wherein said at least one parameter is selected from the group consisting of PEG molecular weight; ratio of hydrophilic to hydrophobic components; CL:LA ratio; and crystallinity.

6. The fibrinogen-based tissue adhesive patch according to claim **4,** wherein said predetermined degradation time is no more than two weeks.

7. The fibrinogen-based tissue adhesive patch according to any one of claims **1 - 6,** wherein said patch is **characterized by** a thickness of about 200 µm.

8. The tissue adhesive patch according to any one of claims **1 - 7,** wherein said fibrinogen sealant additionally comprises at least one additive.

9. The tissue adhesive patch according to claim **8,** wherein said additive is selected from the group consisting of additives for extending the adhesion half-life of said film, pharmaceutically active agents, and analgesics.

10. A method for producing a fibrinogen-based tissue adhesive patch, comprising:
casting a polymer film from PECALA, thereby creating a polymer film **characterized by** a thickness;
softening said polymer film;
placing a fibrinogen sealant comprising less than 8 mg/cm² fibrinogen and less than 20 IU/cm² thrombin on at least one surface of said polymer film; and,
pressing said polymer film until at least a portion of said fibrinogen sealant is incorporated into the surface of said polymer film;
wherein said PECALA comprises PEG having a molecular weight of between 3000 and 3500 and a CL:LA ratio of 34:2.

11. A method for preparing a fibrinogen-based tissue adhesive patch, comprising:
heating to a predetermined temperature a work surface in connection with a source of vacuum;
engaging said vacuum to said work surface;
applying a solution of PECALA to said work surface;
adjusting a polymer blade to a predetermined height above said work surface;
spreading said solution of PECALA over said work surface with said polymer blade;
evaporating said solvent, thereby creating a non-permeable biocompatible polymer film **characterized by** a thickness;
heating said work surface above said softening temperature;
spreading over said polymer film a powder comprising a fibrinogen sealant, said fibrinogen sealant comprising less than 8 mg/cm² fibrinogen and less than 20 IU/cm² thrombin;
placing over said polymer film a top release sheet over said powder and polymer film;
applying pressure to said top release sheet so as to at least partially incorporate said powder into said polymer film, thereby forming a film of adhesive patch material;
removing said top release sheet from said film of adhesive patch material;
releasing said vacuum;
cooling said work surface to room temperature; and,
removing said adhesive patch material from said work surface;
wherein said PECALA comprises PEG having a molecular weight of between 3000 and 3500 and a CL:LA ratio of 34:2.

12. The method according to either one of claims **10** or **11,** wherein at least one condition selected from the group consisting of:
said fibrinogen sealant comprises less than 8 mg/cm² fibrinogen, less than 20 IU/cm² thrombin, and CaCl₂; and,
said thickness is about 200 µm;
is true.

13. The fibrinogen-based tissue adhesive patch according to any one of claims **1 - 9** for use in the treatment of a leak of fluid into or out of a body part.

14. The fibrinogen-based tissue adhesive patch for use according to claim **13,** wherein said leak of fluid is selected from the group consisting of arterial bleeding; organ tissue bleeding; bile anastomosis; cerebrospinal fluid leak; dura leak; and air leak in damaged lung tissue.

15. The fibrinogen-based tissue adhesive patch for use according to claim **13,** wherein said treatment comprises applying a tissue adhesive patch by manually pressing said patch on the surface of said body part.

## Patentansprüche

1. Gewebeklebepflaster auf Fibrinogenbasis, wobei das Klebepflaster folgendes umfasst:
einen Träger, der aus einer Folie hergestellt ist, die aus einem biokompatiblen Polyethylenglycol-Caprolacton-Lactid(PEG-CL-LA)-Dreiblockcopolymer (PECALA) hergestellt ist; und
ein Fibrinogen-Versiegelungsmittel, umfassend weniger als 8 mg Fibrinogen pro cm² und weniger als 20 IE Thrombin pro cm², eingearbeitet in den Träger aus biokompatiblem Polymer;
wobei das PECALA PEG mit einer Molekülmasse zwischen 3000 und 3500 und einem CL:LA-Verhältnis von 34:2 umfasst.

2. Gewebeklebepflaster auf Fibrinogenbasis nach Anspruch 1, wobei das Fibrinogen-Versiegelungsmittel etwa 2 mg Fibrinogen pro cm² und 10 IE Thrombin pro cm² umfasst.

3. Gewebeklebepflaster auf Fibrinogenbasis nach Anspruch 1, wobei das Fibrinogen-Versiegelungsmittel weniger als 8 mg Fibrinogen pro cm², weniger als 20 IE Thrombin pro cm² und CaCl₂ umfasst.

4. Gewebeklebepflaster auf Fibrinogenbasis nach einem der Ansprüche 1 bis 3, wobei mindestens ein Parameter, der das PECALA kennzeichnet, derart gewählt ist, dass das Pflaster eine vorab festgelegte Abbauzeit aufweist.

5. Gewebeklebepflaster auf Fibrinogenbasis nach Anspruch 4, wobei der mindestens eine Parameter aus der Gruppe bestehend aus PEG-Molekülmasse; Verhältnis von hydrophilen zu hydrophoben Komponenten; CL:LA-Verhältnis; und Kristallinität ausgewählt ist.

6. Gewebeklebepflaster auf Fibrinogenbasis nach Anspruch 4, wobei die vorab festgelegte Abbauzeit nicht mehr als zwei Wochen beträgt.

7. Gewebeklebepflaster auf Fibrinogenbasis nach einem der Ansprüche 1 bis 6, wobei das Pflaster durch eine Dicke von etwa 200 µm gekennzeichnet ist.

8. Gewebeklebepflaster nach einem der Ansprüche 1 bis 7, wobei das Fibrinogen-Versiegelungsmittel zusätzlich mindestens einen Zusatzstoff umfasst.

9. Gewebeklebepflaster nach Anspruch 8, wobei der Zusatzstoff aus der Gruppe bestehend aus Zusatzstoffen zum Verlängern der Adhäsionshalbwertszeit der Folie, pharmazeutischen Wirkstoffen und Analgetika ausgewählt ist.

10. Verfahren zum Herstellen eines Gewebeklebepflasters auf Fibrinogenbasis, umfassend:
Gießen einer Polymerfolie aus PECALA, wodurch eine Polymerfolie erzeugt wird, die durch eine Dicke gekennzeichnet ist;
Erweichen der Polymerfolie;
Aufbringen eines Fibrinogen-Versiegelungsmittels umfassend weniger als 8 mg Fibrinogen pro cm² und weniger als 20 IE Thrombin pro cm² auf mindestens einer Oberfläche der Polymerfolie; und
Pressen der Polymerfolie, bis mindestens ein Teil des Fibrinogen-Versiegelungsmittels in die Oberfläche der Polymerfolie eingearbeitet ist;
wobei das PECALA PEG mit einer Molekülmasse zwischen 3000 und 3500 und einem CL:LA-Verhältnis von 34:2 umfasst.

11. Verfahren zum Herstellen eines Gewebeklebepflasters auf Fibrinogenbasis, umfassend:
Erwärmen einer Arbeitsfläche in Verbindung mit einer Vakuumquelle auf eine vorbestimmte Temperatur;
Anlegen des Vakuums an die Arbeitsfläche;
Auftragen einer PECALA-Lösung auf die Arbeitsfläche;
Einstellen einer Polymerklinge auf eine vorbestimmte Höhe über der Arbeitsfläche;
Verteilen der PECALA-Lösung auf der Arbeitsfläche mit der Polymerklinge;
Verdampfen des Lösungsmittels, wodurch eine nichtpermeable biokompatible Polymerfolie erzeugt wird, die durch eine Dicke gekennzeichnet ist;
Erwärmen der Arbeitsfläche auf über die Erweichungstemperatur;
Verteilen eines Pulvers umfassend ein Fibrinogen-Versiegelungsmittel auf der Polymerfolie, wobei das Fibrinogen-Versiegelungsmittel weniger als 8 mg Fibrinogen pro cm² und weniger als 20 IE Thrombin pro cm² umfasst;
Platzieren einer oberen Trennblatts über der Polymerfolie über dem Pulver und der Polymerfolie;
Ausüben von Druck auf das obere Trennblatt, um das Pulver zumindest teilweise in die Polymerfolie einzuarbeiten, wodurch eine Folie aus Klebepflastermaterial gebildet wird;
Entfernen des oberen Trennblatts von der Folie aus Klebepflastermaterial;
Aufheben des Vakuums;
Abkühlen der Arbeitsfläche auf Raumtemperatur; und
Entfernen des Klebepflastermaterials von der Arbeitsfläche;
wobei das PECALA PEG mit einer Molekülmasse zwischen 3000 und 3500 und einem CL:LA-Verhältnis von 34:2 umfasst.

12. Verfahren nach einem der Ansprüche 10 oder 11, wobei mindestens eine Bedingung, ausgewählt aus der Gruppe bestehend aus:
das Fibrinogen-Versiegelungsmittel umfasst weniger als 8 mg Fibrinogen pro cm², weniger als 20 IE Thrombin pro cm² und CaCl₂; und
die Dicke beträgt etwa 200 µm;
zutreffend ist.

13. Gewebeklebepflaster auf Fibrinogenbasis nach einem der Ansprüche 1 bis 9 zur Verwendung bei der Behandlung einer Flüssigkeitsleckage in oder aus einem Körperteil.

14. Gewebeklebepflaster auf Fibrinogenbasis zur Verwendung nach Anspruch 13, wobei die Flüssigkeitsleckage aus der Gruppe bestehend aus arterieller Blutung; Organgewebeblutung; Gallenanastomose; Liquorleckage; Dura-Leckage; und Luftleckage in geschädigtem Lungengewebe ausgewählt ist.

15. Gewebeklebepflaster auf Fibrinogenbasis zur Verwendung nach Anspruch 13, wobei die Behandlung das Auftragen eines Gewebeklebepflasters durch manuelles Drücken des Pflasters auf die Oberfläche des Körperteils umfasst.

## Revendications

1. Timbre adhésif tissulaire à base de fibrinogène dans lequel ledit timbre adhésif comprend :
un support formé par un film à base d'un copolymère (PECALA) biocompatible triséquencé de polyéthylène glycol-caprolactone-lactide (PEG-CL-LA) ; et,
une colle de fibrinogène comprenant au moins 8 mg/cm² de fibrinogène et moins de 20 UI/cm² de thrombine incorporés dans ledit support polymère biocompatible ;
dans lequel ledit PECALA comprend du PEG ayant un poids moléculaire compris entre 3 000 et 3 500 et un rapport CL/LA de 34/2.

2. Timbre adhésif tissulaire à base de fibrinogène selon la revendication 1, dans lequel ladite colle à base de fibrinogène comprend environ 2 mg/cm² de fibrinogène et environ 10 UI/cm² de trombine.

3. Timbre adhésif tissulaire à base de fibrinogène selon la revendication 1, dans lequel ladite colle à base de fibrinogène comprend moins de 8 mg/cm² de fibrinogène, moins de 20 UI/cm² de thrombine, et du CaCl₂.

4. Timbre adhésif tissulaire à base de fibrinogène selon l'une quelconque des revendications 1 à 3, dans lequel au moins un paramètre caractérisant ledit PECALA est sélectionné pour doter ledit timbre d'une durée de dégradation prédéterminée.

5. Timbre adhésif tissulaire à base de fibrinogène selon la revendication 4, dans lequel ledit au moins un paramètre est sélectionné dans le groupe constitué par le poids moléculaire du PEG ; le rapport des composants hydrophiles sur les composants hydrophobes ; le rapport CL/LA ; et la cristallinité.

6. Timbre adhésif tissulaire à base de fibrinogène selon la revendication 4, dans lequel ladite durée de dégradation prédéterminée n'est pas supérieure à deux semaines.

7. Timbre adhésif tissulaire à base de fibrinogène selon l'une quelconque des revendications 1 à 6, dans lequel ledit timbre est **caractérisé par** une épaisseur d'environ 200 µm.

8. Timbre adhésif tissulaire selon l'une quelconque des revendications 1 à 7, dans lequel ladite colle de fibrinogène comprend en outre au moins un additif.

9. Timbre adhésif tissulaire selon la revendication 8, dans lequel ledit additif est sélectionné dans le groupe constitué des additifs pour prolonger la demi-vie d'adhésion dudit film, des agents pharmaceutiquement actifs, et des analgésiques.

10. Procédé de production d'un timbre adhésif tissulaire à base de fibrinogène, comprenant :
le coulage d'un film polymère de PECALA, créant ainsi un film polymère **caractérisé par** une épaisseur ;
le ramollissement dudit film polymère ;
le fait de placer une colle de fibrinogène comprenant moins de 8 mg/cm² de fibrinogène et moins de 20 UI/cm² de thrombine sur au moins une surface dudit film polymère ; et
la compression dudit film polymère jusqu'à ce qu'au moins une partie de ladite colle de fibrinogène soit incorporée dans la surface dudit film polymère ;
dans lequel ledit PECALA comprend du PEG ayant un poids moléculaire compris entre 3 000 et 3 500 et un rapport CL/LA de 34/2.

11. Procédé de préparation d'un timbre adhésif tissulaire à base de fibrinogène, comprenant :
le chauffage à une température prédéterminée d'une surface de travail en relation avec une source de vide ;
la mise en prise dudit vide avec ladite surface de travail ;
l'application d'une solution de PECALA à ladite surface de travail ;
l'ajustement d'une lame polymère à une hauteur prédéterminée au-dessus de ladite surface de travail ;
l'étalement de ladite solution de PECALA sur ladite surface de travail avec ladite lame polymère ;
l'évaporation dudit solvant, créant ainsi un film polymère biocompatible non perméable **caractérisé par** une épaisseur ;
le chauffage de ladite surface de travail au-dessus de ladite température de ramollissement ;
l'étalement sur ledit film polymère d'une poudre comprenant une colle de fibrinogène, ladite colle de fibrinogène comprenant moins de 8 mg/cm² de fibrinogène et moins de 20 UI/cm² de thrombine ;
le placement sur ledit film polymère d'une feuille antiadhésive supérieure sur ladite poudre et ledit film polymère ;
l'application de pression à ladite feuille antiadhésive supérieure pour au moins partiellement incorporer ladite poudre dans ledit film polymère, formant ainsi un film d'un matériau de timbre adhésif ;
le retrait de ladite feuille antiadhésive supérieure dudit film de matériau de timbre adhésif ;
la suppression dudit vide ;
le refroidissement de ladite surface de travail à température ambiante ; et
le retrait dudit matériau de timbre adhésif de ladite surface de travail ;
dans lequel ledit PECALA comprend du PEG ayant un poids moléculaire compris entre 3 000 et 3 500 et un rapport CL/LA de 34/2.

12. Procédé selon l'une ou l'autre des revendications 10 ou 11, dans lequel au moins une condition sélectionnée dans le groupe constitué par :
ladite colle de fibrinogène comprend moins de 8 mg/cm² de fibrinogène, moins de 20 UI /cm² de thrombine, et du CaCl₂ ; et,
ladite épaisseur est d'environ 200 µm ;
est vraie.

13. Timbre adhésif tissulaire à base de fibrinogène selon l'une quelconque des revendications 1 à 9 pour son utilisation dans le traitement d'une fuite de fluide dans ou hors d'une partie corporelle.

14. Timbre adhésif tissulaire à base de fibrinogène pour son utilisation selon la revendication 13, dans lequel ladite fuite de fluide est sélectionnée dans le groupe constitué d'une hémorragie artérielle ; d'une hémorragie d'un tissu organique ; d'une anastomose biliaire ; d'une fuite de liquide céphalorachidien ; d'une fuite à travers la dure mère ; et d'une fuite d'air dans un tissu pulmonaire endommagé.

15. Timbre adhésif tissulaire à base de fibrinogène pour son utilisation selon la revendication 13, dans lequel ledit traitement comprend l'application d'un timbre adhésif tissulaire par compression manuelle dudit timbre sur la surface de ladite partie corporelle.
